(19) Europäisches Patentamt
European Patent Office
Office européen des brevets

(11) **EP 3 375 879 A1**

(12) **EUROPEAN PATENT APPLICATION**

(43) Date of publication:
**19.09.2018 Bulletin 2018/38**

(51) Int Cl.:
*C12N 15/64* (2006.01)          *A61K 48/00* (2006.01)
*C12P 19/34* (2006.01)          *C12N 15/85* (2006.01)

(21) Application number: **18161814.1**

(22) Date of filing: **14.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.03.2017 US 201762470997 P**

(71) Applicants:
• **Baylor College Of Medicine**
  **Houston, 77030 (US)**
• **The Board of Regents of The University of Texas System**
  **Austin, TX 78701 (US)**

(72) Inventors:
• **ZECHIEDRICH, E., Lynn**
  **Houston, TX 77225 (US)**
• **FOGG, Jonathan, Marcus**
  **Houston, TX T77030 (US)**
• **WANG, Qian**
  **Austin, TX 78701 (US)**
• **PETTITT, B., Montgomery**
  **Austin, TX 78701 (US)**

(74) Representative: **Barker Brettell LLP**
**100 Hagley Road**
**Edgbaston**
**Birmingham B16 8QQ (GB)**

(54) **TARGETING MINIVECTORS TO SPECIFIC TISSUE USING SHAPE**

(57) Minivectors having defined, non-transient shapes as determined by sequence are provided, along with uses in the targeting delivery of such minivectors to target tissues for preferential gene delivery. Method of designing and making shaped minivectors are also provided.

FIGURE 9

**Description**

**PRIOR RELATED APPLICATIONS**

This application claims priority to US Serial Number 62/470,997, filed March 14, 2017, and incorporated by reference in its entirety for all purposes.

**FEDERALLY SPONSORED RESEARCH STATEMENT**

This invention was made with government support under RO1A1054830, R56AI054830, A1054830, R01GM115501, GM115501, and GM066813 awarded by the National Institutes of Health and CNS-1338192 awarded by the National Science Foundation. The government has certain rights in the invention.

**FIELD OF THE DISCLOSURE**

The disclosure generally relates to methods of targeting minivectors to specific tissues or organs using specific shapes of the minivectors themselves. Method of designing and making shaped minivectors are also provided.

**BACKGROUND OF THE DISCLOSURE**

One of the most important objectives in gene therapy is the development of highly safe and efficient vector systems for gene transfer to eukaryotic cells. Initially, viral-based vector systems were used, most commonly retroviruses or adenoviruses, to deliver the desired gene. Other viruses used as vectors include adeno-associated viruses, lentiviruses, pox viruses, alphaviruses, and herpes viruses. The main advantage of virus-based vectors is that they have evolved to physically deliver a genetic payload into cells and this can be readily exploited. The efficiency of delivery into cells is generally much higher than e.g., plasmid DNAs.

Viral-based vectors can have disadvantages however. Viruses can usually infect more than one type of cell and can infect healthy cells as well as diseased cells. Another danger is that the new gene might be inserted in the wrong location in the genome, possibly causing cancer or other problems. This has already occurred in clinical trials for X-linked severe combined immunodeficiency (X-SCID) patients. In addition, there is a small chance that viral DNA could unintentionally be introduced into the patient's reproductive cells, thus producing changes that may be passed on to children. Another concern is the possibility that transferred genes could be overexpressed, producing so much of the added protein as to be harmful. Finally, the viral vector could cause an immune reaction or could be transmitted from the patient to other individuals or even into the environment.

Plasmids could potentially be used instead of viral based vectors. Plasmid are much less efficient at entering cells, but have some utility in basic research because they are straightforward to generate and isolate. In fact, clinical trials using intramuscular injection of a naked DNA plasmid have occurred with some success. Unfortunately, expression has been very low in comparison to other methods of transfection.

Numerous studies have shown that the bacterial backbone in plasmids results in immunity problems and reduction of transgene expression. Based on this observation, minicircle DNA vectors were developed. Minicircles are small (<4 kb) circular plasmid derivatives that are almost completely devoid of bacterial sequences (such as the antibiotic resistance genes and origin of replication). They have been applied as transgene carriers for the genetic modification of mammalian cells, with the advantage that, since they contain no bacterial DNA sequences, they are less likely to suffer from the well documented, but little understood, silencing of transgene expression that often occurs when the transgene is carried on a vector containing bacterial sequences. Bacterial DNA sequences may also potentially illicit immune responses and therefore minicircle vectors are less likely to induce an immune reaction.

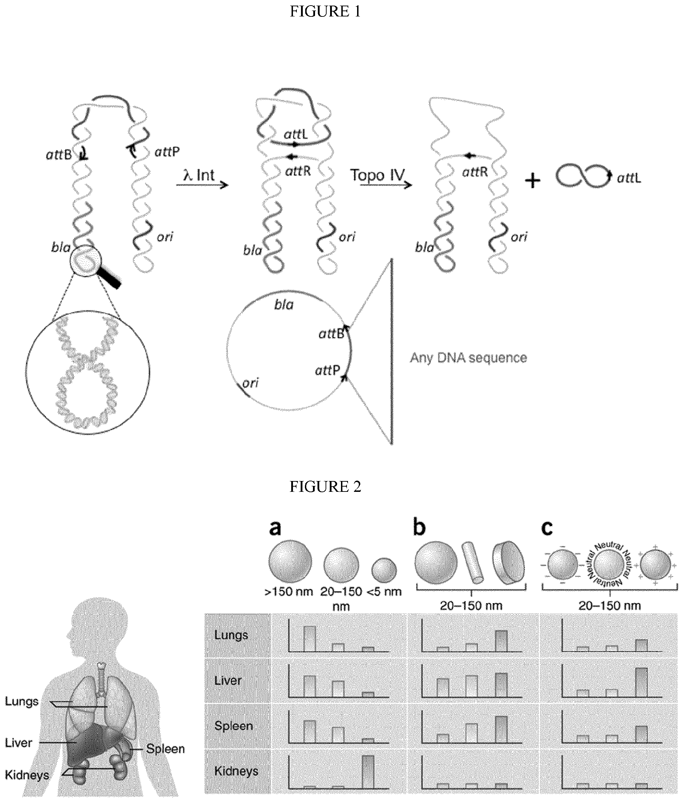

Their preparation usually follows this basic procedure, outlined below and shown in **FIG. 1:**

1) production of a 'parental plasmid' (bacterial plasmid containing the transgene or other sequence to be delivered, flanked by target sites for site-specific recombination, *att*L and *att*R) in *E. coli.*

2) induction of the site-specific recombinase λ-integrase (Int), thereby excising bacterial DNA sequences via strand exchange at the target sites, excising the bacterial sequences and leaving a minimal vector containing only the desired sequences to be delivered. Other recombinases can be used.

3) purification of the resulting minicircle (vehicle for the highly efficient modification of the recipient cell) and removal of contaminants such as any unrecombined parent plasmid and the excised large circle, the other product of recombination containing the bacterial sequences. If the different DNA species are of sufficiently different size they can be readily separated by size-exclusion chromatography (gel-filtration), yielding a highly pure preparation of minicircle.

The purified minicircle can be transferred into recipient cells by transfection or lipofection and into a differentiated tissue by, for instance, jet injection.

Conventional minicircles lack an origin of replication, so they do not replicate within the target cells and the encoded genes will disappear as the cell divides (which can be either an advantage or disadvantage depending on whether the application demands persistent or transient expression). A promising development is nonviral self-replicating minicircles, which owe this property to the presence of a scaffold/ matrix attachment region (S/MAR)-Element. The S/MAR element ensures that the minicircle are maintained episomally, with once per cell cycle replication, synchronized with the host genome. Self-replicating minicircles hold great promise for the systematic modification of stem cells and will significantly extend the potential of their plasmid precursor forms.

Up to now, minicircle vectors are produced by recombinases such as: λ integrase, φ C31 integrase, C recombinase and FLP recombinase, with specific advantages and disadvantages for each. Several studies have demonstrated that minicircles are safe and episomal vectors that enhance transgene expression extent and duration of transgene expression *in vivo* and *in vitro.*

An at-first seemingly unrelated application of DNA minicircles is for study of the structure and dynamics of supercoiled DNA and it's interactions with proteins. For these applications it is beneficial to make the minicircles as small as feasible, ideally just a few hundred base-pairs. Larger DNAs, such as plasmids, or even many so-called "minicircles" are several kbp in size. It is very difficult to study the structure of DNA with these substrates because the important subtleties of structure are averaged out using conventional means of structure determination (e.g. NMR, cryoEM or cryoET etc). Also important is that the small size of minicircles allows researchers to perform all-atom simulations, something that is impossible with larger DNAs because the computational power required scales nonlinearly with DNA size.

The use of DNA minicircles, less than 1,000 bp, is promising, but they were initially difficult to produce and purify in significant quantity. Additionally, their use was limited by a difficulty to produce minicircles containing a physiological topology *i.e.*, supercoiling. Without an origin of replication, closed topologically constrained circles are not efficiently produced in large quantities *in vivo.* Site-specific recombination is inhibited when the recombination sites are closely spaced, and intermolecular recombination, between sites on two separate plasmids becomes more favorable leading to multimeric products (Fogg 2006). An alternative approach commonly used is the circularization of linear molecules to form minicircles. However, yields are low and intermolecular ligation contaminants are prevalent when the short linear DNA molecules necessary for generating minicircles are used.

US7622252 overcomes the topology problem by transforming the plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA minicircle of less than about 1 kb in size. Then, the catenated products are decatenated with an endonuclease, and supercoiled minicircles and nicked circles are recovered. The nicked circles can also be supercoiled in the presence of intercalators and ligase. Minicircle vectors generated by this method are called "minivectors."

Although minivector technology is promising, there remains a need for improved delivery mechanisms. The ideal method would allow systemic delivery of a gene, such as one contained in a minivector, yet it would still allow targeting to the organ or tissue of interest.

## SUMMARY OF THE DISCLOSURE

The importance of the *size* of targeted, spherical drug carriers has been explored and the guiding principles are reasonably well understood. However, particle *shape* has recently emerged as an equally important parameter in determining the *in vivo* journey of particles.

Overall, increasing a particles aspect ratio (AR) has been found to decrease macrophage phagocytosis efficiency, due to changes of local curvature, which promoted cellular phagocytosis. In general, higher AR particles were found to increase circulation time by avoiding filtration by the spleen and sequestration in the liver when compared to spherical analogs, although this was dependent on the overall physical dimensions, particle matrix, and flexibility. Non-spherical particles were also shown to positively influence margination, with disks and rods improving migration to endothelial walls, although this effect was restricted to micron-sized particles under physiological blood flow conditions. Additionally, both drug release and active targeting through ligand-cell interactions were directly related to surface area, which depends on particle shape. Finally, cellular internalization rates and cellular mechanisms were shown to be loosely dependent on particle geometry, but were confounded by differences in exact particle geometry, matrix, and surface chemistry. See **FIG. 2.**

Although very promising in its ability of allowing tissue targeting based in part on shape, until now it has not been possible to control the shape of DNA molecules to take advantage of this phenomena. Minivectors, because of their small size, have unrivaled utility, not only for the delivery of therapeutic sequences, but also for the study and understanding of how DNA supercoiling and sequence affects the shape of the molecule. We have previously shown how supercoiling can modify the shape of the minivector, how certain sequences can affect where bending occurs, and how bending at one site can affect the localization of supercoiling-induced bending elsewhere in the molecule.

We are now taking this further by exploring how we can introduce specific sequences that will modify the overall shape of the DNA. In conjunction with modeling efforts, we have developed DNA minivectors with a variety of different shapes and will demonstrate their preferential targeting. Although work is ongoing, this opens up the amazing possibility of preparing minivectors that are preferentially targeted to tissues and organs of interest based on shape, thus allowing systemic delivery of the minivectors. This is an area with tremendous potential and is only possible because of the benefits of the minivectors, notably their small size and the ability to incorporate any sequence.

As used herein, a "minivector" is a double stranded circular DNA lacking a bacterial origin of replication and an antibiotic selection gene, and having a size of about 100 bp up to about 5 kbp. It is usually obtained by site-specific recombination of a parent plasmid to eliminate plasmid sequences outside of the recombination sites. It contains, for example, a nucleic acid molecule with merely the transgene expression cassette (including promoter and a nucleic acid sequence of interest, wherein the nucleic acid sequence may be, for example, a template for e.g., homology-directed repair, alteration, or replacement of the targeted DNA sequence, silencing sequences, and, importantly, no bacterial-originated sequences.

Purity levels of MiniVectors™ are typically much higher than a minicircle preparation and there is usually, by agarose gel electrophoresis analysis, no detectible contamination by catenanes, the other circular recombination product, nor the parent plasmid. For clarity, the incidence of MiniVector dimers and sometimes higher multimer MiniVectors, as described in above, is sometimes as much as 5-10%. These do not constitute contaminants and they are merely double (or triple, *etc.*) the desired therapy. Nonetheless, as good diligence requires, an extra gel filtration step typically separates higher multimers from unit-sized MiniVector if needed.

It is important to note that when analyzing topology for MiniVectors less than 1 kb, even high percentage agarose gels lack the resolution to separate supercoiled from relaxed MiniVector or linear DNA of the same size, and we recommend polyacrylamide gel electrophoresis. Individual topoisomers may even be resolved and isolated using this technique. For optimum separation of topoisomers we recommend the addition of 10 mM $CaCl_2$ (or 10 mM $MgCl_2$) to the running buffer. For more details of electrophoresis with MiniVector DNA, please see Fogg (2006).

As used herein, "decatenating" can be performed, for example, by treating the catenated product with a restriction endonuclease that only cleaves the larger DNA circle and not the DNA minicircle. However, it is preferred that decatenation occur with residual topoisomerase IV or similar activity, not a restriction enzyme, as this can be done *in vivo* using the endogenous bacterial topoisomerases and does not require any additional restriction sites or enzyme coding sequences.

Site-specific recombination can be performed *in vivo* or *in vitro* using purified components, but *in vivo* methods are preferred, especially using topoisomerase IV inhibition with fluoroquinones. Topoisomerase IV has both decatenation and supercoil relaxation activities. Inhibition of topoisomerase IV both slows decatenation and increases the supercoiling level in the cell. Integrase-mediated site-specific recombination is strongly supercoiling dependent, therefore increasing supercoiling by inhibiting topoisomerase IV also stimulates recombination. Topoisomerase IV has enough residual decatenation activity, even in the presence of fluoroquinolones, to eventually decatenate the products.

The linearized product is separated from the supercoiled DNA MiniVectors by any of a number of methods known in the art, e.g., agarose gel electrophoresis, polyacrylamide gel electrophoresis, column chromatography, density centrifugation, and the like.

As used herein, "shape" encompasses the basic geometric shapes, such as star, rod, disc, and the like, as well as including features such as aspect ratio, local surface roughness, features in all three-dimensions, varied surface curvatures, the potential for creative and diverse biomimicry, numbers of surface appendages, extreme geometries, etc.

As used herein, "a defined geometric shape" means that the minivector has a particular geometric shape, such as e.g., a rod, a star, a hexagon, a cube or rhomboid, a rod, or a tetrahedron, that is non-transient, e.g., is retained in solution and *in vivo* at a high equilibrium concentration (>50%) with respect to other possible shapes. It expressly excludes linear or nicked DNAs that freely change shape in solution or ordinary supercoiled DNAs lacking a non-transient shape imposed thereon. Furthermore, the shape is a function of the DNA sequence, and is not externally imposed thereon, e.g., by histones, capsid proteins, or micelles, and the like.

As used herein, when we say that > 50% of said minivectors have a specific shape, we mean that when measured or visualized we see that more than half of the vectors have the same shape, although that shaped may be viewed from different angles.

As used herein, "non-transient" means that the shape is retained in solution and *in vivo* (unless nicked) at a high equilibrium value with respect to other shapes. Transient shapes, by contrast include the various forms that a simple circular or linear DNA can take in solution, such as random linear DNA shapes, circles that are twisted and distorted in one or more directions and may have a broad range of shapes in equilibrium.

As used herein, a "non-transient shape" is to be assessed by electron microscopy that > 50% of the minivectors demonstrate the desired shape, preferably with an equilibrium population of > 75%, > 85%, or better.

As used herein a "rod" is a generally cylindrical shape that is elongated, and has an aspect ratio (AR) of > 5.

As used herein a "microrod" is a rod that is at least 1 micron long in the long axis. Particles with diameters on the order of microns (interestingly about the same size as a platelet) preferentially displace to the cell free layer (CFL) in the presence of red blood cells (RBCs), while smaller particles do not experience this enhanced localization.

A "nanorod" by contrast is of length in the long axis < 1 micron. Nanorods may accumulate significantly in the spleen.

As used herein, a "star" shape has a plurality of generally evenly sized and distributed projections, e.g., six armed stars have been shown to be preferentially delivered to pulmonary tissue.

The use of the word "a" or "an" when used in conjunction with the term "comprising" in the claims or the specification means one or more than one, unless the context dictates otherwise.

The term "about" means the stated value plus or minus the margin of error of measurement or plus or minus 10% if no method of measurement is indicated.

The use of the term "or" in the claims is used to mean "and/or" unless explicitly indicated to refer to alternatives only or if the alternatives are mutually exclusive.

The terms "comprise", "have", "include" and "contain" (and their variants) are open-ended linking verbs and allow the addition of other elements when used in a claim.

The phrase "consisting of' is closed, and excludes all additional elements.

The phrase "consisting essentially of' excludes additional material elements, but allows the inclusions of non-material elements that do not substantially change the nature of the invention, such as instructions for use, buffers, and the like.

The invention includes any one or more of the following embodiment(s), in any combination(s) thereof:

A minivector, said minivector being a double stranded, circular DNA encoding a sequence of interest and lacking a bacterial origin of replication and lacking an antibiotic resistance gene, wherein > 50% of said minivectors have a specific shape.

A minivector, said minivector being a double stranded, circular DNA encoding a sequence of interest and lacking a bacterial origin of replication and lacking an antibiotic resistance gene, wherein > 50% of said minivectors have a defined, non-transient shape as determined visually.

A minivector, said minivector being a double stranded, circular DNA encoding a sequence of interest and lacking a bacterial origin of replication and lacking an antibiotic resistance gene, wherein > 50% of said minivectors have a defined, non-transient shape as determined visually from 3-D projection of minivector structure derived from electron cryo-tomography data.

Any minivector herein described, said shape having an aspect ratio (AR) of > 20. or having an AR above 20, and major axes of 9 $\mu$m and 27 $\mu$m respectively.

Any minivector herein described, being hexagonal, an elliptical disc, a star, a discoid, a racquet, a microrod or a nanorod.

A method of gene therapy, comprising administering any minivector herein described to a patient, said shape preferentially directing said minivector to a target tissue, and preferentially expressing said sequence of interest in said target tissue.

A minivector of defined 3D shape, said 3D shape having been produced by controlling a level of supercoiling of said minivector by nicking said minivector and religating in the presence of intercalators or HmfB, wherein a torsional strain associated with negative supercoiling (underwinding) leads to localized disruptions in a helical structure at one or more hyperflexible sites determined by a sequence of said minivector, thereby producing said shape.

A minivector of defined 3D shape, said 3D shape having been produced by controlling a level of supercoiling said minivector and by designing a sequence of said minivector using the following equation to predict mechanical correlations in bending at base pair i:

$$(i + N/Nv)\%N, (i + 2N/Nv)\%N \ldots (i + (Nv\text{-}1)*N/Nv)\%N,$$

where N is the number of the total base pair,

Nv is the number of bend locations, and

% represents the modulo operation and * is multiplication.

The following abbreviations are used herein:

| ABBREVIATION | TERM |
| --- | --- |
| AR | Aspect ratio |
| BCC | Bent vertices correlation coefficient |
| CFR | Cell free layer |
| CryoET | Cryo-electron tomography |
| EPR | enhanced permeability and retention |
| FACS | fluorescence-activated cell sorting |
| Lk | Linking number |
| MC | Minicircle |
| PP | Parental Plasmid |
| RBC | Red blood cells |
| Rg | Radii of gyration |
| VMD | Visual Molecular Dynamics |

## BRIEF DESCRIPTION OF THE DRAWINGS

**FIG. 1.** Generation of minivector DNA by $\lambda$-integrase mediated site-specific recombination. Parent plasmid containing the sequence to be delivered, flanked by *att*L and *atti*R, target sites for recombination. The parent plasmid is propagated in the special *E. coli* bacterial host strain, LZ54, harboring $\lambda$-integrase (iInt) under the control of the temperature sensitive cl857 repressor. When the cells have reached a suitable density, expression of Int is switched on by temperature switch. Recombination results in a catenated product containing the minicircle. The products are decatenated, either by endonuclease cleavage of the large circle deletion product, or by topoisomerase IV mediated unlinking, subsequent to the removal of topoisomerase inhibitor following the cell harvest. The deletion product, containing the undesired bacterial sequences is removed, yielding pure, supercoiled minicircle product.

**FIG. 2.** Size and shape considerations for delivery of particles to tissues, from Blanco (2015). (a) Spherical particles, including gold nanoparticles, liposomes and polymeric micelles/nanoparticles can vary in size and display disparate *in vivo* fates. Large rigid particles with diameters >2,000 nm accumulate readily within the spleen and liver, as well as in the capillaries of the lungs. Nanoparticles in the range of 100-200 nm have been shown to extravasate through vascular fenestrations of tumors (the EPR effect) and escape filtration by liver and spleen. As size increases beyond 150 nm, more and more nanoparticles are entrapped within the liver and spleen. Small-sized nanoparticles (<5 nm) are filtered out by the kidneys. (b) Novel 'top-down' and 'bottom up' fabrication techniques have enabled the exploration of different geometries of nanoparticles, including cylindrical and discoidal shapes, which have been shown to exhibit pronounced effects on pharmacokinetics and biodistribution. Different nanoparticle shapes exhibit unique flow characteristics that substantially alter circulating lifetimes, cell membrane interactions and macrophage uptake, which in turn affect biodistribution among the different organs, (c) Charge of nanoparticles stemming from distinct surface chemistries influences opsonization, circulation times and interaction with resident macrophages of organs comprising the MPS, with positively charged particles more prone to sequestration by macrophages in the lungs, liver and spleen. Neutral and slightly negatively charged nanoparticles have longer circulation lifetimes and less accumulation in the organs of the MPS93. In both b and c, the size of the nanoparticles is assumed to range from 20-150 nm. Individual panels represent *in vivo* fates of nanoparticles, taking into account singular design parameters of size, shape and surface charge independent of one another, and for this reason, respective scales vary from one panel to the next. *In vivo* biodistribution will undoubtedly vary based on the interplay of the above parameters.

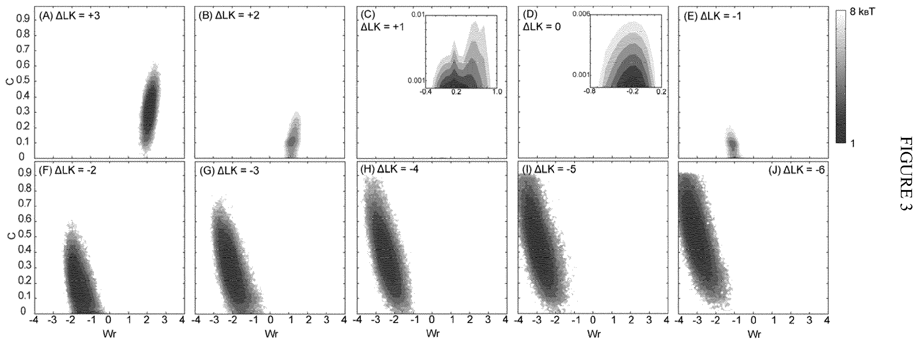

**FIG. 3A-J.** Free energy landscape of DNA minicircles under different torsional stress as a function of the percentage of helix to helix contacts, C, and writhe, Wr. For (C) and (D), inset figures were added to zoom in the free energy minimum. The unit of free energy is kBT where T = 310K and kB is Boltzmann's constant. Free energy is shown from darkest color (lowest) to white (highest).

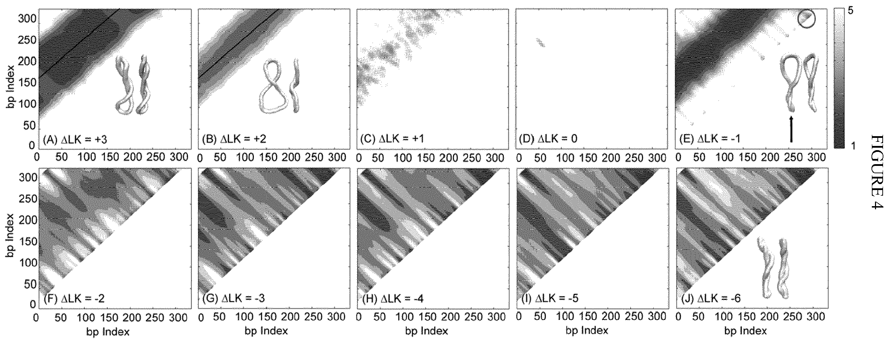

**FIG. 4A-J.** Free energy, in units of kBT (T = 310K), of contact formation between base pairs shown from dark (lowest) to white (highest). The black guide line through the dark color in (A) and (B) is y = x + 168. A few representative structures are shown for two views differed by 90°. In (E), the structure represents the typical conformation inside the circle region. The arrow shows the position of base-pair opening and base unstacking.

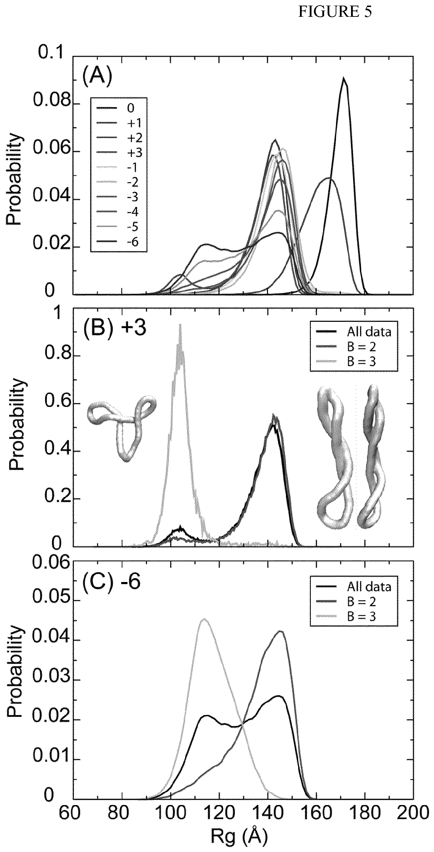

**FIG. 5A-C.** Distribution of the radii of gyration for DNA minicircles under torsional stress. (A) Radii of gyration (Rg) of DNA minicircles under all the various torsional stresses; (B) Rg of DNA minicircles at ΔLk = +3 for either all data, when B = 2 (two sharply bent vertices) or when B = 3 (three bent vertices); (C) same as (B) but for the ΔLk = -6 minicircles.

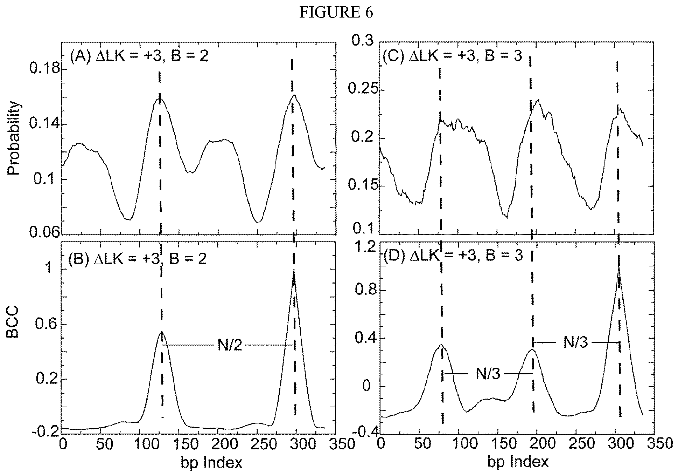

**FIG. 6A-D.** Demonstration of positive mechanical correlations along the DNA minicircle contour length. (A) Probability of becoming the site of a bent vertex for each base pair for ΔLk = +3, B = 2; (B) Bent vertices correlation coefficient (BCC) between bp 293 and other base pairs, for the ΔLk = +3 minicircles, B = 2; (C) Probability of becoming a bent vertex for each base pair for minicircle ΔLk = +3, B = 3 and (D) Bent vertices correlation coefficient between bp 305 and other base pairs, for minicircles of ΔLk = +3, B = 3. N is the total number of the base pair, 336. An example for the structure of B = 3 is shown on the left in **FIG. 5B.** Dashed lines represent the idealized placement of bent vertices given the first bend's placement.

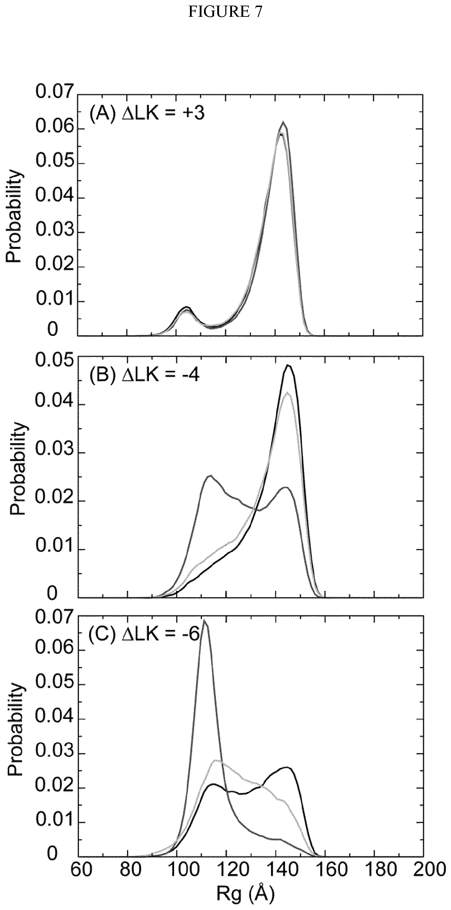

**FIG. 7A-C.** Distribution of the radii of gyration for the DNA minicircle and minicircles containing the original sequence (black), sequence S2 (grey) or S3 (light grey) under different torsional conditions: (A) ΔLk = +3, (B) ΔLk = -4 and (C) ΔLk = -6.

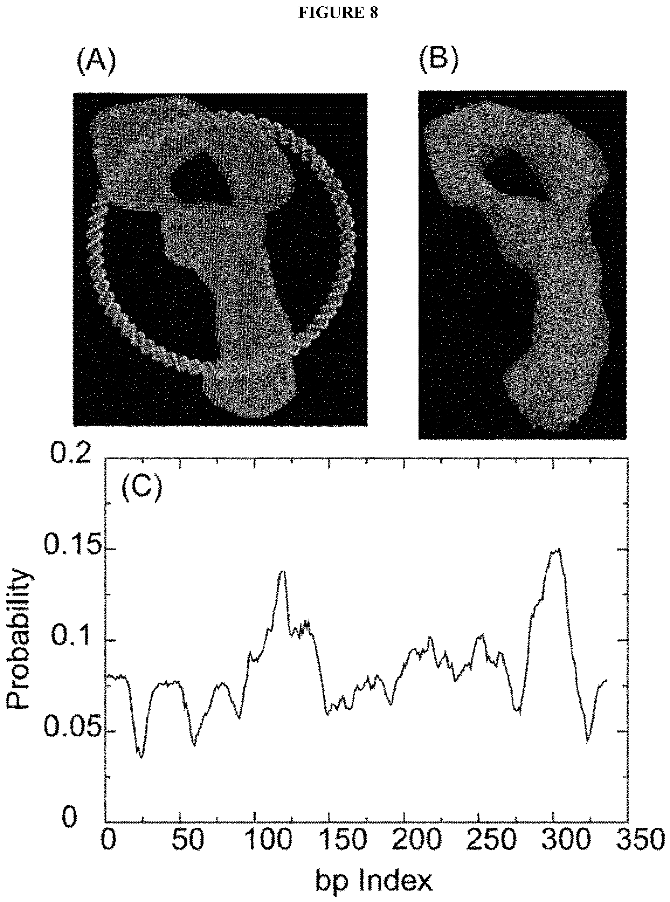

**FIG. 8A-C.** Refinement of cryo-ET density map for a structure with ΔLk = -2: (A) An initial structure of a DNA minicircle (the nucleosides are in red and the phosphates are in green) overlaid on cryo-ET image (cyan). (B) Superimposition of 250 random structures (red) obtained from the constraint fitting simulation overlaid over cryo-ET image (cyan). (C) The probability of each base pair to become a bending edge.

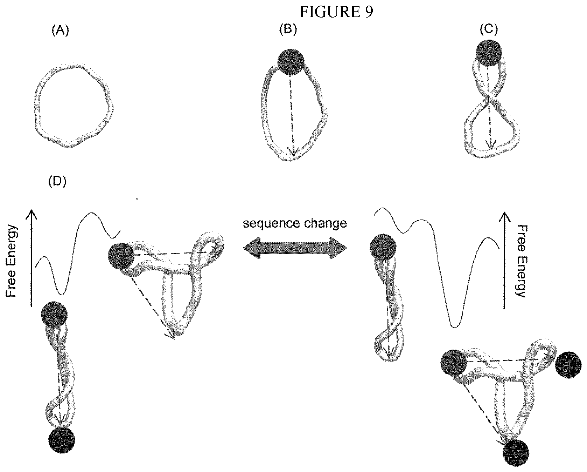

**FIG. 9A-D.** Conformational equilibria of a DNA minicircle under torsional stress. Torsional stress gradually increases from (A) to (D). Grey dots mark the most thermodynamic unstable segment along the minicircle. Black dots mark other thermodynamic unstable segments. Grey dash lines represent a strong mechanical correlation. For (D), a dynamic equilibrium exists between two structures: two vertices and three vertices. The free energy difference between these two structures shown schematically depends on the sequence (location of the black dots).

## DETAILED DESCRIPTION

The disclosure provides novel minivectors having defined, non-transient 3D structures or shapes and methods of using the shaped minivectors to preferentially target their delivery to particular tissues based on that shape. Also included are mathematical methods of designing specific shapes, which are then executed in a DNA form, usually by DNA synthesis.

Sequence dependence of the conformational distribution of DNA minicircles under various levels of torsional stress was previously an unsolved problem, which prevented the manufacture and use of shaped minivectors.

By nicking minivectors and religating in the presence of intercalators or HmfB we can precisely control the level of supercoiling. The torsional strain associated with negative supercoiling (underwinding) leads to localized disruptions in the helical structure of DNA. These localized disruptions modify the properties of DNA, generating a hyperflexible site. This, coupled with the tendency of supercoiled DNA to writhe to relieve torsional strain, results in the formation of a DNA bend at the site at which the helical structure is disrupted. We have found that DNA minicircle topoisomers have multiple bending vertices under high torsional stress and that the positions of these bending vertices are determined by the sequence and a positive mechanical correlation along the sequence. We show herein that simulations and theory are able to provide sequence-specific information about individual DNA minicircles, and our 3D structure predictions are confirmed by cryo-electron tomography (cryo-ET).

Our results provide proof of concept that the conformational distribution of minicircles under torsional stress can be controlled by modifying the sequence and supercoiling, which has important implications for using minicircle DNA for gene therapy. We can now design minivectors to have particular sizes and shapes, and use those features to preferentially target tissues for gene delivery.

In this work, we employed coarse-grained simulations and theoretical analyses to model DNA minicircles under a wide range of torsional stress, including the highly positively and highly negatively supercoiled regimes not previously simulated. These new simulations provided a means to relate the structures observed in our previous work with the DNA sequence. We found that a thermodynamically unstable segment of the minicircle cooperates with a mechanical correlation from the stress of circularization to determine minicircle structure. With this new information, we were able to determine how the sequence, coupled with supercoiling, can modify the final 3D structure. We used these findings to computationally design a new three lobed structure (star). Overall, our improved coarse-grained modeling qualitatively explains how DNA sequence defines the three-dimensional structure of supercoiled DNA minicircles.

We used two different simulation procedures herein. The first was done to sample the full possible conformational distributions allowed by the model. This unconstrained simulation included the 336 bp minicircle sequence, but did not include cryo-ET structural data. The resulting structural distributions were then compared with the distribution of minicircle conformations seen by cryo-ET. The second procedure was a constrained simulation. We performed these simulations with a bias potential to restrict the results to the experimental data.

## UNCONSTRAINED SIMULATION

We simulated the 336 bp DNA minicircle of sequence SED ID NO: 1:

TTTATACTAACTTGAGCGAAACGGGAAGGGTTTTCACCGATATCACCGAAACGCGC

GAGGCAGCTGTATGGCATGAAAGAGTTCTTCCCGGAAAACGCGGTGGAATATTTCG

TTTCCTACTACGACTACTATCAGCCGGAAGCCTATGTACCGAGTTCCGACACTTTCA

TTGAGAAAGATGCCTCAGCTCTGTTACAGGTCACTAATACCATCTAAGTAGTTGATT

CATAGTGACTGCATATGTTGTGTTTTACAGTATTATGTAGTCTGTTTTTTATGCAAA

ATCTAATTTAATATATTGATATTTATATCATTTTACGTTTCTCGTTCAGCTTT.

The coarse-grained force field model used was originally developed by the Louis group. In this model, the parameters of hydrogen-bonding and stacking interactions were fit to reproduce the melting temperature of the simulated DNA sequences. This model successfully predicts structural alterations in DNA oligomers under mechanical stress.

DNA minicircle conformation can change with salt. Because the original Louis model (Sulc 2012, Wang 2014) did not consider the effects of salt, we modified the original model by approximating the electrostatic interactions between two charged beads in presence of ions as a Debye-Hückel potential:

$$V_{ij} = \frac{\gamma q_i q_j}{4\pi\varepsilon_0\varepsilon_r r} e^{-r/\sqrt{\varepsilon_0\varepsilon_r k_B T/2e^2 I}}$$

where i and j are the phosphate atoms and r is the separation between them. $\varepsilon_0$ is the permittivity of free space and $\varepsilon_r$ is the relative dielectric constant (set to 80). I is the ionic strength of the system, $\gamma = 0.7$ was an empirical fit obtained in previous work to match the DNA melting temperatures under the various different salt conditions.

We set the ionic strength to 0.1 M and the temperature to 310 K at an effective 1 atm of pressure. Newtonian dynamics were applied and the Andersen thermostat method was used to maintain the temperature.

Linking number, $Lk$, is the number of times one DNA strand wraps around the other. The Watson-Crick strands of a relaxed 336 bp minicircle wrap around each other 32 times, defining $Lk_0$ (i.e. having no supercoiling). $\Delta Lk$ is the difference between $Lk$ under a given torsional stress and $Lk_0$. We simulated three different positively supercoiled (overwound) topoisomers: $Lk$ = 33 ($\Delta Lk$ = +1), 34 (+2) and 35 (+3). We also simulated six negatively supercoiled (underwound) topoisomers $Lk$ = 26 to 31 ($\Delta Lk$ = -6 to -1) (Irobalieva, 2015). For each topoisomer, $Lk$ is a topological invariant that satisfies the following relationship:

$$Lk = Wr + Tw$$

where $Wr$ is writhe, measured as the average number of crossings of the minicircle with itself seen from all projections. $Tw$ is twist, measured along the helical axis, and refers to the number of helical repeats in the DNA.

In silico, for each linking number Lk, we first built a planar circular minicircle without any crossing points (Wr = 0). Under this circumstance, Lk = Tw. The twisting angle in degrees between each stacking base pair, $\theta$, satisfies the following relationship:

$$336 \times \theta = 360 \times Lk$$

We next performed simulations, each initiated from the starting planar minicircle. *Lk* did not change in the simulation but the partition between *Wr* and *Tw* varied with *Lk,* which was directly observed in the simulations.

For each linking number, the system was equilibrated for 15 ns (because this is a coarse-grained model the time scale is approximate). After the equilibration, each simulation was performed for 60 ns more and data were recorded every 150 ps. Each simulation was repeated 240 times with different random starting velocities, making the overall sampling time 14 $\mu$s for each linking number. The root-mean-square deviation of the minicircle coordinates for the 240 trajectories and an example of a single trajectory are not shown, but indicate that the minicircle can be fully equilibrated within 60 ns simulation for a single trajectory and the conformational space can be well sampled with 240 trajectories. We found that even with a coarse-grained model, a large number of trajectories (in our case, 240) from different initial conditions were essential in order to sample the conformational space. The density maps produced were processed in VMD.

## CONSTRAINED SIMULATIONS

These simulations were performed with an introduced bias potential that restrained results to conformations found in the cryo-ET minicircle density data from Irobaliava 2015. This protocol was developed for all-atomistic simulations and we previously applied it to our coarse-grained DNA model (Wang 2015). The bias potential from electron microscopy, $V_{EM}$, was added to the original DNA Hamiltonian as follows:

$$V_{EM}(x, y, z) = \begin{cases} 0 \\ \varepsilon \times (-\varphi(x, y, z)) & if\,(\varphi < \varphi_c) \\ & else \end{cases}$$

$$\varepsilon = 0.05E \text{ where } E = 4.14x10^{-20}\,J.$$

The value of $\varphi$ was obtained from the cryo-ET density (Irobalieva, 2015) as follows: the tomogram was divided onto a grid with dimension $45 \times 100 \times 80$ angstroms. The bin size was set to 4.52 Å/pixel to match the sampling of the subvolume. In the experiment, the density of each grid point was measured and recorded as $\rho$. $\rho$ was scaled with the unit $\sigma$, the standard deviation of $\rho$. In the simulation $\varphi$ of each cell grid point was modeled as the value of $\rho$ at a corresponding point, which was read directly from the cryo-ET density, $\rho$ ranges from $- 0.8\sigma$ to $+0.8\sigma$ in the experiment. However, relatively small $\rho$ may correspond to the noise that needs to be screened before simulation. It is difficult to infer an accurate cut-off value, $\varphi_c$ for the screening process. Here, we set an empirical cut-off, $\varphi_c$ = 0.27, to screen the noise in the experiment, because the isosurface of the experimental surface map does not change significantly when $\varphi_c$ < 0.27.

The initial structure of the simulation was set to an unbiased planar circle with random orientations relative to the center of the experimental density. The center of mass velocity of the DNA minicircle model was set to zero. Otherwise, the same simulation protocol as described above was performed for the biased refinement simulations. Each biased simulation set consisted of 1,200 repeats of 300 ns, giving a total of 360 $\mu$s. The probability of each base pair in the sequence to bend was determined.

## CONFORMATION OF MINICIRCLES UNDER TORSIONAL STRESS

Cryo-ET revealed an ensemble of 3D structures of DNA minicircles of various *Lk* (Irobalieva, 2015). We tested how well our coarse-grained model predicted the observed conformations- and in so doing were able to assign DNA sequence to the cryo-ET structures. We first simulated the distribution of structures for each topoisomer *de novo.* We used a force field model at ambient temperature. Thus, we expected to sample a distribution of possible structures corresponding to those found in the flash frozen solution used for the cryo-ET. We showed that this model could be used to assign DNA sequence within the experimental cryo-ET structures in the cases with either sharp kinks or bends.

The conformational distribution of the DNA minicircle is reflected in the free energy map as a function of the writhe (*Wr*) and the fraction of contact formations "*C*" (**FIG. 3**). A contact formation is defined as an event where the distance between any backbone bead in one base pair and any backbone bead in another base pair is less than 3 σ (σ = 8.52Å). *Wr* measures the average number of crossings of the minicircle seen from all vantage points, while the y-axis, *C*, indicates how tightly intertwined the circle is. As a general trend, it is clear that for both positive and negative supercoiling, when the torsional stress increases, both *Wr* and *C* increase, indicating a higher probability of the DNA becoming more intertwined with itself. In addition, the conformational distribution becomes broader. Both *Wr* and *C* fluctuate more under high torsional stress than low torsional stress. This result is explained by an increase of bp opening (Table 1) and DNA crossing points that accompanied high torsional stress, which increased the number of possible minicircle conformations. The distribution of the radius of gyration ($R_g$) was also broader under high torsional stress, as shown in **FIG. 5A.**

Table 1.

| Probability of the DNA minicircle having at least one base pair opening | | | | | | | | |
|---|---|---|---|---|---|---|---|---|
| ΔLk | 0 | +1 | +2 | +3 | -1 | -2 | -3 | -4 and above |
| Probability | 0 | 0 | 0.6% | 4.5% | 10.3% | 85.0% | 99.9% | 100% |

As expected, the simulated relaxed DNA minicircle (*ΔLk* = 0) strongly peaked at *Wr* ~ 0 and *C* ~ 0 (**FIG. 3D**). The dominant structure was a nearly planar circle. Roughly 5% of the *Lk* = 32 circles were concave (not shown). When *ΔLk* = +1, *C* and *Wr* slightly increased (**FIG. 3C**) and ~42% became nonplanar (not shown). With increased positive twist (*ΔLk* = +2), the probability of nonzero writhe increased. When *ΔLk* = +2, the dominant (average) structure shifted to the region with *Wr* = 1 and *C* ~ 10% (**FIG. 3B**), indicating that there is a high probability of a single crossing of the minicircle in the circle. The position of this crossing is identified by the base pair contact map (**FIG. 4B**). Each data point describes the free energy, which is calculated as the logarithm of the probability of occurrence of a particular contact formation.

We found that the dominant contact formations for *ΔLk* = +2 (darker areas in **FIG. 4B**) are, on average, between bp *i* and bp *j* satisfying the relation *j* = *x* + 168 (black line in **FIG 4A-B**), which is half of the total length. Because the total bp of this circle is *N* = 336, contact formations are mainly between bp *i* and bp *i+N/2*. In other words, the crossing was found in the center of the circle and evenly split the circle to two roughly equal sub-circles, forming figure-8 shapes. The most common conformation was this figure-8 shape but there were fluctuations from the line *j* = *x* + 168.

When *ΔLk* = +3, the average *Wr* of the probability distribution of structures centered at 2 (**FIG. 3A**). At the same time, the percentage of close duplex contacts, *C*, increased to around 35% corresponding to a more highly intertwined structure. From the base pair contact map (**FIG. 4A**), although the lowest free energy area still can be considered along the line *j* = *x* + 168, the distribution was much broader than it was for the *ΔLk* = +2 minicircle topoisomer (**FIG. 4B**), indicating that a continuous long contact region is formed in the circle between the two crossing points. Such a contact probability formation is reflective of the so-called "handcuff" configurations of Irobalieva (2015) (**FIG. 4A**).

It is clear that the conformation distribution under negative torsional strain is much broader than that under similar positive strain. This result is not surprising given that negative strain favors base pair opening and positive strain does not. Table 1 shows the probability of having at least one base pair opening as a function of *Lk.* These probabilities qualitatively agree with the probability of cleavage by Bal-31 as reported previously (Irobalieva, 2015). For positive strain, even at the most overwound *ΔLk* = +3 topoisomer, there was only a 4.5% probability of base pair opening. In contrast, for negative twist, at *ΔLk* = -1 there is a 10.3% probability to have base pair opening and this probability increased to 100% when *ΔLk* < -3. Base pair opening makes the structure locally very flexible.

For such a small change in linking number, writhe in the *ΔLk* = -1 topoisomer centered at -1.0 and *C* ~ 10% (**FIG. 3E**). This topoisomer has structural features somewhat similar to those of the *ΔLk* = +2 topoisomer. The calculation for the *ΔLk* =_-1_topoisomer (**FIG. 4E**, black arrow), however, indicated base pair opening and base unstacking between two sequential base pairs. As a result, some structures for the *ΔLk* = -1 topoisomer show a so-called racquet configuration.

With increasing negative torsional stress, *Wr* decreased and *C* increased (**FIG. 3F-J**). At *ΔLk* = -6, the most probable *Wr* shifted to -3.0 and the maximum of *C* was up to 90%, indicating that the most probable minicircle conformation is almost totally wound around itself into a rod shape (**FIG. 3J**). The contact map revealed multiple minima (**FIG. 4J**), indicating that the conformational distribution is very broad.

Overall, the topoisomer structures we obtained *in silico* qualitatively matched the experimental results of Irobalieva (2015). Both studies agree that with increasing negative supercoiling, DNA minicircles become more intertwined, evolving from an open circle to a figure-8, racquet, needle, and finally a rod. We calculated the twist of the minicircle (*Tw* = *Lk* - *Wr*) and found that *Tw* increased with *ΔLk* from -6 to +3 (not shown). These results match well to our previous simulations of twist (with no writhe) at these supercoiling levels.

**EFFECT OF TORSIONAL STRESS ON MINICIRCLE COMPACTNESS**

To understand how the degree and direction of supercoiling compacts minicircles, we considered radius of gyration ($Rg$) (**FIG. 5**). $Rg$ is a standard measure for polymers and polyelectrolytes and is experimentally accessible via several techniques. The radius of gyration of DNA minicircles with torsional stress reduced from 170 Å to as small as ~105 Å (**FIG. 5A**). Two distinct $R_g$ maxima, seen for all the negatively supercoiled minicircles and for the most positively supercoiled minicircle, implies two simultaneous populations of structures. We devised a parameter, $B$, that quantifies the number of bent vertices in each minicircle. **FIG. 5B** and **FIG. 5C** show that the radius of gyration distribution for three ensembles: all data, $B = 2$ and $B = 3$. The existence of the ensemble with $B = 3$ accounts for the mechanism of the size reduction of the DNA minicircle with increasing negative supercoiling. $B = 3$ is a characteristic of compact trefoil structures. Such structures have more bends in order to release the high torsional stress. $B = 2$ represents the more extended structures like the figure-8 or the handcuff. Generally speaking, $B$ increases with the increase of torsional stress and the overall global size reduces correspondingly.

**POSITIVE MECHANICAL CORRELATIONS ALONG DNA MINICIRCLES**

Because bends affect the conformation of a minicircle, we explored DNA sequence found at vertices. We uncovered three thermodynamically unstable segments- bp 1-20, bp 104-115 and bp 287-315 with TpA tracts (TA steps are among the most unstable of the base-pair steps (Santalucia (1998) and Olson et al. (1998)). We found these same three DNA segments using a separate analytical thermodynamic method (not shown). One might assume that the multiple possible bent vertices on a DNA minicircle are always more probable at those thermodynamically unstable segments but we found that these were not always the points of bending. The physical connectivity of the sequences in a circle also contribute to which sequences bend. For example, when $\Delta Lk = +3$ and $B = 2$, the two bent vertices are located at bp 297 and bp 127 (**FIG. 6A**), which agrees with the experimental mapping using Bal-31 cleavage (Irobalieva 2105). It is understandable that bp 297 serves as one bend location because bp 297 is within the most thermodynamically unstable segment (not shown). However, the segment near bp 127 is more stable than bp 104-115 or bp 1-20. Therefore, a strong positive mechanical correlation (**FIG. 6B**) between bp 127 and 297 forces bp 127 to bend. Such correlations have been noted previously.

When $B = 3$, there were three bent vertices located at bp 305, 195, and 83 (**FIG. 6C**). Similarly, a positive mechanical correlation among those positions was observed as well (**FIG. 6D**). In addition, the base pair separation among above three positions is close to 1/3 of the total bp number 336. Our results indicate that the positive mechanical correlation resulting from a thermodynamically weak sequence interacting within the circular DNA constraint is one of the important factors affecting the geometric structure distribution under high torsional stress. A general conclusion is that this positive mechanical correlation exists at bp $(i + N/N_v)\%N$, $(i + 2N/N_v)\%N.....$ $(i + (N_v-1)*N/N_v)\%N$, where $N$ is the number of the total base pair, $N_v$ is the number of the bend locations and % represents the modulo operation. Modulating conformation by changing sequence

One of the most thermodynamic unstable segments on the supercoiled minicircle is located at bp 303-315, which is a TpA tract. Intuitively, changing C/G to A/T at or near a predicted vertex should increase bendability. To test this hypothesis, we designed a new sequence, S2. In S2, bp 79-91 was mutated from CTCAAGAAGGGCC to ATAAATAATATAA and bp 191-203 were mutated from ACAATGTCCAGTG to ATAATATATATTA. Such mutations should increase the probability of structures with B = 3 (relative to $B = 2$) and reduce $R_g$. Another sequence: $(AT)_{10}(CG)_{102}(AT)_{10}(CG)_{102}(AT)_{10}(CG)_{102}$ can be considered an extreme case of increasing the probability of $B = 3$. Although at $\Delta Lk = +3$, $R_g$ of all three tested DNA sequences were similar (**FIG. 7**), when $\Delta Lk = -4$ and -6, both S2 and S3 shift to the low $R_g$ region. This result indicates that the conformational distribution of minicircles under torsional stress can be designed.

**ASSIGNING MINICIRCLE SEQUENCE TO CRYO-ET CONFORMATIONS**

Taking advantage of the bends revealed in the cryo-EM density maps, we probed the probability of each base pair to be involved in a bp opening or unstacking to attempt to locate the sequence with respect to the position of kinked or vertex features. **FIG. 8A** shows a cryo-ET density map for $\Delta Lk = -2$. This minicircle is in a racquet conformation and contains a sharp bend at the bottom of the handle of the racquet, as predicted from the analysis above. With a constrained coarse-grained simulation with $\Delta Lk = -2$ we fitted the molecular structure from the cryo-ET density map at the base pair level. The initial structure of such simulation can be seen in **FIG. 8A.** Superimposition of 250 simulated structures are shown in **FIG. 8B.** It is clear that with the constraint, our final average structure is within the experimental cryo-ET density map. Whether these simulations of models actually correspond to the actual sequence alignment is difficult to test precisely experimentally but they agree qualitatively with the previously published Bal-31 cleavage results (Irobalieva, 2015). The probability of each base pair to be localized at the vertex as calculated from our extensive set of initial conditions is shown in **FIG. 8C.** Our fitting result indicates that every base pair has a probability of being at the sharp bend. However, bp 293 and bp 126 have the highest probability. This result qualitatively matches the modeling done without cryo-ET bias constraints (**FIG. 6A**).

In this work, we studied how the conformational distribution of a 336 bp DNA minicircle changes with torsional stress by performing coarse-grained simulations. These simulations allowed for localized structural deformations introducing hyperflexible sites that will facilitate DNA bending. Therefore, the location of the bends in the conformations of supercoiled DNA is modified by sequence. This is an advance of previous work and provides a bridge between the atomic detail provided by all-atom molecular dynamic simulations and the increased conformational sampling that can be achieved by coarse-grained simulations. This, it combines some of the advantages of the two approaches. Not only did we capture the shape characteristics under each torsional strain condition observed in the experiment, but we were also were able to align the DNA sequence in the DNA minicircle (Irobalieva 2015). Our results demonstrate that the conformational distributions of DNA minicircles with supercoiling is highly dependent on the positions of bends, which is dependent on DNA sequence, and on a physical constraint imposed by circularity. The thermodynamically least stable segment along the sequence had the highest probability of bending.

Several analytical methods have been developed to identify the least stable DNA segments more precisely. The location of bends is determined by a mechanical correlation in the minicircle so that the separation between two vertices is half the minicircle. This long-range correlation of circularization previously was observed thermodynamically and kinetically (e.g., Lionberger 2011). With increased torsional stress, crossing juxtapositions began to appear (**FIG. 9C,D**). If there were multiple crossing points (**FIG. 9D**), the structures of the minicircle, dictated by the number of vertices, had broader distribution of conformations. Mechanical correlations dictate that those vertices evenly distribute along the minicircle so that the separation between two adjacent vertices is $N/N_v$ ($N$ is the total bp number and $N_v$ is the number of vertices). The dynamic equilibrium among those structures can be further modulated by the sequence near the potential vertices. Assuming the most thermodynamic unstable bp is $i$, if the bp's near $(i + N/N_v)\%N$, $(i + 2N/N_v)\%N$..... $(i + (N_v-1)*N/N_v)\%N$ (% represents the modulo operation) are also thermodynamically unstable (black dots in **FIG. 9**), then the probability of $N_v$ vertices will increase. This rule can be potentially used to design compact structures of a minicircle, which will increase the transfection efficiency of a DNA minicircle vector in gene therapies.

We developed a way to add sequence registry to the experimental DNA cryo-ET density maps through a geometric penalty function to refine with respect to sequence registry. The model is computationally sufficiently inexpensive to allow not only shape but sequence registry refinement for less than atomic resolution experiments like cryo-ET. Once we understand how DNA sequence influences structure/function of DNA, we may begin to understand how proteins find their sequences, how promoters function, and how DNA metabolism is regulated.

There are limitations in the accuracy of the current coarse-grained model. The intrinsic curvature of the minicircle will affect the length and strength of the mechanical correlations detailed above. In order to describe the correlation more quantitatively, the energy functions of different base pairs need to be modeled accurately, which is an intrinsically difficult task using coarse-grained models. Thus, the results of current work should be considered as qualitative. In addition, besides the intrinsic curvature, architectural proteins can bind to DNA and induce structural deformations. Such effects will be studied in the future.

**FUTURE WORK**

Minivectors, of a few hundred base pairs and with defined shape, will be generated by incorporating DNA sequences that will become bend sites when exposed to the torsional stress associated with negative supercoiling. The first example to be tested will be a simple rod shape. The shape distribution will be determined by electron cryo-tomography. The defined shape will comprise at least 50% of the shape distribution. If necessary, the negative supercoiling will be further increased by nicking the minivector and religating in the presence of intercalators. The experiments will be repeated for additional predicted supercoiling-dependent shapes.

Longer minivectors, of a thousand base pairs or more, with defined shape will also be generated and analyzed as for the smaller minivectors.

Transfection efficiency will be determined by labeling the minivectors with a fluorescent dye and monitoring DNA uptake by human cells in culture using fluorescence-activated cell sorting (FACS). This will be repeated with minivectors of different shapes to determine if there is a preferred shape for cell transfection. Transfection efficiency will also be compared to a minivector of similar size but lacking the sequences that become bend sites.

To further dissect the effect of shape on DNA transfection into human cells in culture we will perform fluorescence microscopy using fluorescently labeled minivectors with defined shape to determine how shape influences nuclear localization. Z stacking will be utilized to precisely determine whether minivectors are in the nucleus or cytoplasm. We anticipate that certain shapes may be more efficient at entering the nucleus.

To confirm that the minivectors with defined shape can still be recognized by the cellular transcription machinery, we will incorporate DNA sequences encoding shRNA against GFP. These experiments will be performed with unlabeled minivectors. Knockdown of GFP in cells expressing GFP in culture will also be measured by FACS. Incorporation of sequences to manipulate shape should not affect transcription efficiency.

Minivectors with defined shape will be generated containing a luciferase gene. The shape will be confirmed by electron cryo-tomography as described above. These minivectors will be injected into the bloodstream of mice by hydrodynamic tail injection and the whole-body distribution of the minivectors in live mice will be determined by monitoring the *in vivo* bioluminescence from the luciferase gene (a mutant version that does not get secreted so it will stay where it is expressed) following injection of the D-luciferin substrate. We anticipate that the shape will affect which organs are transfected by minivectors. At the completion of the bioluminescence study, mice will be sacrificed, dissected, and individual organs imaged to assay for the presence of minivector. The experiments will be repeated with minivectors of different shapes, in addition to a control without added bend sites, to determine the effect of shape on biodistribution.

Through modulating of DNA shape, we may also modify the radius of gyration of the minivector DNA. A more compact minivector with lower radius of gyration will be less susceptible to the hydrodynamic shearing associated with aerosolization. We have shown previously that the size, supercoiling level, and shape of circular DNA vectors strongly affect the survival during nebulization (Catanese *et al.* 2011). In the absence of vehicle, DNA vectors above 2,000 bp are very susceptible to shear-induced degradation during nebulization. By modulating the shape and radius of gyration we anticipate that we may be able to generate DNA vectors (of sizes longer than 2,000 bp) that are able to withstand nebulization. We will generate minivectors with different shapes and compare their ability to survive nebulization following the protocols described in Catanese *et al.* 2011.

The present invention is exemplified with respect to DNA minicircles of SEQ ID NO.1 and variants thereof. However, this is exemplary only, and the invention can be broadly applied to minivector DNA of a variety of shapes and sequences.

The following references are incorporated by reference in their entirety for all purposes.

Blanco, E., et al. (2015) Box 2: Nanoparticle rational design implementation for overcoming delivery barriers, Nature Biotechnology 33: 941-951

Darquet et al. (1999) "Minicircle: an improved DNA molecule for in vitro and in vivo gene transfer," Gene Therapy, 6: 209-218.

Fish, M.B., et al. (2015) Emergence and Utility of Nonspherical Particles in Biomedicine, Ind Eng Chem Res. 54(16): 4043-4059.

Fogg, J.M., et al. (2006) Exploring writhe in supercoiled minicircle DNA. J. Phys.-Condes. Matter, 18: S145-S159.

Irobalieva, R.N., et al. (2015) Structural diversity of supercoiled DNA. Nat. Commun., 6: 10.

Lionberger, T.A., et al, (2011) Cooperative kinking at distant sites in mechanically stressed DNA. Nucleic Acids Res., 39: 9820-9832.

Olson, W.K., et al. (1998) DNA sequence-dependent deformability deduced from protein-DNA crystal complexes.. Proc. Natl. Acad. Sci. U.S.A., 95: 11163-11168.

SantaLucia, J. (1998) A unified view of polymer, dumbbell, and oligonucleotide nearest-neighbor thermodynamics. Proc. Natl. Acad. Sci. U.S.A., 95: 1460-1465.

Sulc, P., et al. (2012) Sequence-dependent thermodynamics of a coarse-grained DNA model. J. Chem. Phys., 137, 14.

Wang, Q. and Pettitt, B.M. (2014) Modeling DNA thermodynamics under torsional stress. Biophys. J., 106, 1182-1193.

Wang, Q., et al. (2015) Twist-induced defects of the p-ssp7 genome revealed by modeling the cryo-em density. J. Phys. Chem. B, 119: 4937-4943.

US20150376645, US20140056868, 61/653,279, filed May 30, 2012, Supercoiled minivectors as a tool for DNA repair, alteration and replacement

US8460924, US8729044, US9267150, US20110160284, US20120302625, US20130316449, 61/252,455, filed Oct.

16, 2009, Supercoiled minivectors™ for gene therapy applications

US7622252, US20070020659, 60/689,298, filed Jun. 10, 2005, Generation of minicircle DNA with physiological supercoiling

US20060211117 Methods of making minicircles

WO1994009127 Supercoiled minicircle DNA as a unitary promoter vector

WO2002083889 Methods for the production of minicircles

**Claims**

1. A minivector, said minivector being a double stranded, circular DNA encoding a sequence of interest, and lacking a bacterial origin of replication and lacking an antibiotic resistance gene, wherein > 50% of said minivectors have a specific equilibrium shape.

2. A minivector, said minivector being a double stranded, circular DNA encoding a sequence of interest, and lacking a bacterial origin of replication and lacking an antibiotic resistance gene, wherein > 50% of said minivectors have a defined, non-transient shape as determined visually.

3. A minivector, said minivector being a double stranded, circular DNA encoding a sequence of interest, and lacking a bacterial origin of replication and lacking an antibiotic resistance gene, wherein > 50% of said minivectors have a defined, non-transient shape as determined visually from 3-D projection of minivector structure derived from electron cryo-tomography data.

4. The minivector of any of claims 1-3, said shape having an aspect ratio (AR) of > 20.

5. The minivector of any of claims 1-3, having an ARs above 20, and major axes of 9 $\mu$m and 27 $\mu$m respectively.

6. The minivector of any of claims 1-3, being hexagonal.

7. The minivector of any of claims 1-3, being an elliptical disc.

8. The minivector of any of claims 1-3, being a star.

9. The minivector of any of claims 1-3, being discoid.

10. The minivector of any of claims 1-3, being a racquet.

11. The minivector of any of claims 1-4, being a microrod.

12. The minivector of any of claims 1-4, being a nanorod.

13. A method of gene therapy, comprising administering the minivector of any of claims 1- 12. to a patient, said shape preferentially directing said minivector to a target tissue, and preferentially expressing said sequence of interest in said target tissue.

14. A minivector of defined 3D shape, said 3D shape having been produced by controlling a level of supercoiling of said minivector by nicking said minivector and religating in the presence of intercalators or HmfB, wherein a torsional strain associated with negative supercoiling (underwinding) leads to localized disruptions in a helical structure at one or more hyperflexible sites determined by a sequence of said minivector, thereby producing said shape.

15. A minivector of defined 3D shape, said 3D shape having been produced by controlling a level of supercoiling said minivector and by designing a sequence of said minivector using the following equation to predict mechanical correlations in bending at base pair $i$:

$$(i+ N/N_v)\%N,\ (i+ 2N/N_v)\%N\ldots(i + (N_v\text{-}1)*N/N_v)\%N,$$

where $N$ is the number of the total base pair,
$N_v$ is the number of bend locations, and
% represents the modulo operation and * is multiplication.

$$(i+ N/N_v)\%N,\ (i+ 2N/N_v)\%N\ldots(i + (N_v\text{-}1)*N/N_v)\%N,$$

FIGURE 1

FIGURE 2

FIGURE 3

FIGURE 4

FIGURE 5

FIGURE 6

FIGURE 7

**FIGURE 8**

(A)

(B)

(C)

FIGURE 9

(A)  (B)  (C)

(D)

sequence change

Europäisches Patentamt
European Patent Office
Office européen des brevets

## EUROPEAN SEARCH REPORT

Application Number

EP 18 16 1814

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | IROBALIEVA, R.N. ET AL.: "Structural diversity of supercoiled DNA", NAT. COMMUN., vol. 6, 8440, 12 October 2015 (2015-10-12), pages 1-10, XP002782270, DOI: 10.1038/ncomms9440 | 1-12,14 | INV. C12N15/64 A61K48/00 C12P19/34 C12N15/85 |
| Y | * page 7, column 2, paragraph 2; figures 1,2,3,5 * | 13 | |
| Y | US 2013/085173 A1 (ZECHIEDRICH E LYNN [US] ET AL) 4 April 2013 (2013-04-04) * claims 1-31 * | 13 | |
| A | WANG QIAN ET AL: "Sequence Affects the Cyclization of DNA Minicircles.", 17 March 2016 (2016-03-17), THE JOURNAL OF PHYSICAL CHEMISTRY LETTERS 17 MAR 2016, VOL. 7, NR. 6, PAGE(S) 1042 - 1046, XP002782271, ISSN: 1948-7185 * the whole document * | 1-14 | |
| Y,D | US 2015/376645 A1 (ZECHIEDRICH E LYNN [US] ET AL) 31 December 2015 (2015-12-31) * claims 26-35 * | 13 | TECHNICAL FIELDS SEARCHED (IPC) C12N A61K C12P |
| X,P | WANG QIAN ET AL: "Influence of DNA sequence on the structure of minicircles under torsional stress", 27 July 2017 (2017-07-27), NUCLEIC ACIDS RESEARCH, VOL. 45, NR. 13, PAGE(S) 7633-7642, XP002782272, ISSN: 0305-1048(print) * the whole document * | 1-15 | |

-/--

The present search report has been drawn up for all claims

| Place of search | Date of completion of the search | Examiner |
|---|---|---|
| Munich | 21 June 2018 | Seroz, Thierry |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 18 16 1814

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NAOME A, LAAKSONEN A, VERCAUTEREN D: "A Coarse-Grained Simulation Study of the Structures, Energetics, andDynamics of Linear and Circular DNA with Its Ions", JOURNAL OF CHEMICAL THEORY AND COMPUTATION, vol. 11, 1 May 2015 (2015-05-01), pages 2813-2826, XP002782273, * the whole document * | 1-15 | |
| A | SUTTHIBUTPONG THANA ET AL: "Long-range correlations in the mechanics of small DNA circles under topological stress revealed by multi-scale simulation", 2 November 2016 (2016-11-02), NUCLEIC ACIDS RESEARCH, VOL. 44, NR. 19, PAGE(S) 9121-9130, XP002782274, ISSN: 0305-1048(print) * the whole document * | 1-15 | |
| A | WANG QIAN ET AL: "Modeling DNA Thermodynamics under Torsional Stress", BIOPHYSICAL JOURNAL, vol. 106, no. 5, 31 January 2014 (2014-01-31), pages 1182-1193, XP028625412, ISSN: 0006-3495, DOI: 10.1016/J.BPJ.2014.01.022 * the whole document * | 1-15 | TECHNICAL FIELDS SEARCHED (IPC) |
| A | ZHENG XIAOZHONG ET AL: "Theoretical Analysis of Disruptions in DNA Minicircles", BIOPHYSICAL JOURNAL, vol. 96, no. 4, February 2009 (2009-02), pages 1341-1349, XP002782275, ISSN: 0006-3495 * the whole document * | 1-15 | |

-/--

The present search report has been drawn up for all claims

| Place of search | Date of completion of the search | Examiner |
|---|---|---|
| Munich | 21 June 2018 | Seroz, Thierry |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WANG, Q. ET AL.: "Twist-induced defects of the p-ssp7 genome revealed by modeling the cryo-em density", J. PHYS. CHEM. B, vol. 119, 2015, pages 4937-4943, XP002782276, * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 21 June 2018 | Seroz, Thierry |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 16 1814

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2018

| Patent document cited in search report | Publication date | Patent family member(s) | Publication date |
|---|---|---|---|
| US 2013085173 A1 | 04-04-2013 | NONE | |
| US 2015376645 A1 | 31-12-2015 | AU    2013267350 A1 | 29-01-2015 |
| | | BR 112014030007 A2 | 27-06-2017 |
| | | CA    2876860 A1 | 05-12-2013 |
| | | EP    2854866 A1 | 08-04-2015 |
| | | JP  2015523860 A | 20-08-2015 |
| | | KR  20150027756 A | 12-03-2015 |
| | | US  2014056868 A1 | 27-02-2014 |
| | | US  2015376645 A1 | 31-12-2015 |
| | | WO  2013181440 A1 | 05-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62470997 B
- US 7622252 B
- US 20150376645 A
- US 20140056868 A
- US 61653279 B
- US 8460924 B
- US 8729044 B
- US 9267150 B
- US 20110160284 A

- US 20120302625 A
- US 20130316449 A
- US 61252455 B
- US 20070020659 A
- US 60689298 B
- US 20060211117 A
- WO 1994009127 A
- WO 2002083889 A

### Non-patent literature cited in the description

- **BLANCO, E. et al.** Box 2: Nanoparticle rational design implementation for overcoming delivery barriers. *Nature Biotechnology,* 2015, vol. 33, 941-951
- **DARQUET et al.** Minicircle: an improved DNA molecule for in vitro and in vivo gene transfer. *Gene Therapy,* 1999, vol. 6, 209-218
- **FISH, M.B. et al.** Emergence and Utility of Nonspherical Particles in Biomedicine. *Ind Eng Chem Res.,* 2015, vol. 54 (16), 4043-4059
- **FOGG, J.M. et al.** Exploring writhe in supercoiled minicircle DNA. *J. Phys.-Condes. Matter,* 2006, vol. 18, S145-S159
- **IROBALIEVA, R.N. et al.** Structural diversity of supercoiled DNA. *Nat. Commun.,* 2015, vol. 6, 10
- **LIONBERGER, T.A. et al.** Cooperative kinking at distant sites in mechanically stressed DNA. *Nucleic Acids Res.,* 2011, vol. 39, 9820-9832

- **OLSON, W.K. et al.** DNA sequence-dependent deformability deduced from protein-DNA crystal complexes. *Proc. Natl. Acad. Sci. U.S.A.,* 1998, vol. 95, 11163-11168
- **SANTALUCIA, J.** A unified view of polymer, dumbbell, and oligonucleotide nearest-neighbor thermodynamics. *Proc. Natl. Acad. Sci. U.S.A.,* 1998, vol. 95, 1460-1465
- **SULC, P. et al.** Sequence-dependent thermodynamics of a coarse-grained DNA model. *J. Chem. Phys.,* 2012, vol. 137, 14
- **WANG, Q. ; PETTITT, B.M.** Modeling DNA thermodynamics under torsional stress. *Biophys. J.,* 2014, vol. 106, 1182-1193
- **WANG, Q. et al.** Twist-induced defects of the p-ssp7 genome revealed by modeling the cryo-em density. *J. Phys. Chem. B,* 2015, vol. 119, 4937-4943